# EUROPEAN PATENT SPECIFICATION

(11) **EP 1 930 042 B1**
(45) Date of publication and mention of the grant of the patent: **24.11.2010**
(21) Application number: 07254252.5
(22) Date of filing: 26.10.2007
(51) Int. Cl.: A61M 16/08, A61M 16/00

(54) **Apparatus having a water trap of detectable form, and method**
Vorrichtung mit einer Wasserauffangvorrichtung mit feststellbarer Form und Verfahren
Appareil doté d'un piège à eau de forme détectable et procédé

(30) Priority: 09.12.2006 DE 102006058164
(43) Date of publication of application: 11.06.2008
(73) Proprietor: Dräger Medical AG & Co. KG, 23558 Lübeck (DE)
(72) Inventor: Peter, Gerd, 23562 Lübeck (DE); Maxeiner, Thomas, 23570 Lübeck (DE)
(74) Representative: Greenwood, John David

(56) References cited:
- EP-A- 1 579 884
- WO-A-00/45884
- DE-C1- 19 901 590
- US-B1- 6 294 997
- US-B1- 6 337 631

## Description

The invention relates to an apparatus having a water trap. The apparatus has a gas sensor and is designed to pass a flow of sample gas through the water trap and feed it to the gas sensor. The water trap is designed to be disconnectably connected to the apparatus. The apparatus has a receiving means for connection to the water trap. The apparatus is for example a monitor device for analysing gas or a respirator apparatus.

A water trap for a flow of sample gas is known from DE 199 015 90 C1. The water trap has a housing which can be disconnectably connected to a holder. The housing has a tank for receiving liquid.

Document WO 00/45884 discloses a similar apparatus.

An apparatus connected to a patient circuit and to absorber means is known from EP 1 579 884 A1. The apparatus has information transfer means and electronic means and an electronically programmable transponder containing information. The transponder is connected to the absorber means. The information transfer means are designed to communicate with the transponder, and the electronic means are designed to read out procedures from a base of information which is received from an anaesthesia/ventilating system and, via the information transfer means, from the transponder, which latter is connected to the absorber means.

With water traps known from the prior art, such too as the water trap which is known from DE 19901590 C1 for example, there is the problem that germs may collect in the water trap, and in particular in a container belonging to the water trap. There is therefore a restriction on the time for which the water trap can be used and it is recommended that use be made of the water trap for only a limited time. In practice, the operator of a respirator apparatus notes down the date on which a new water trap is connected to the respirator apparatus. On the expiry of a predetermined interval of time, which is preset by, for example, the manufacturer of a water trap, the water trap has to be exchanged by the operating personnel and replaced with a new one.

The object underlying the invention is to improve an apparatus in respect of the handling of the water trap.

This object is achieved as claimed by an apparatus according to the present invention in which the water trap has a radio-frequency marker. The apparatus has a radio-frequency sensing device having a sensing range for the radio-frequency marker, which radio-frequency sensing device is designed to sense the radio-frequency marker in the sensing range and to generate a marker signal which represents an item of marker information relating to the radio-frequency marker. The apparatus is designed to be controlled as a function of the marker signal. In this way, the apparatus is advantageously able to sense a state where the water trap is connected to the apparatus. The apparatus is designed, to sense a period of time for which the state of the water trap being connected has lasted.

In a preferred embodiment, the apparatus has a time counter which is connected at least indirectly to the radio-frequency sensing device and which is designed to generate, as a function of the marker signal and in particular of at least one presence of the marker signal, an interval of time which represents a period of time for which the water trap has been connected to the apparatus, and to compare the interval of time with a predetermined interval of time to expiry and to generate a result of the comparison. The apparatus is further designed to generate, as a function of the result of the comparison, an expiry signal which represents an expiry of the predetermined interval of time to expiry. The apparatus is designed to be controlled as a function of the expiry signal. It can advantageously be ensured in this way that the water trap is not connected to the apparatus for a period of time longer than the period of time corresponding to the interval of time to expiry.

The apparatus may for example be controlled as a function of the expiry signal in such a way as to generate an audio or visual warning signal as a function of the expiry signal and to reproduce this warning signal. For this purpose, the apparatus may have for example an indicator unit or light-signal emitter which is designed to generate the warning signal.

In an advantageous embodiment, the apparatus is designed to be at least partly enabled or disenabled as a function of the marker signal. As a further preference, the apparatus is designed to be at least partly enabled or disenabled as a function of the expiry signal. It can advantageously be ensured in this way that a water trap is not connected to the apparatus for more than a period of time corresponding to the interval of time to expiry. The apparatus may for example be at least partly enabled or disenabled in such a way that predetermined controls of the apparatus are enabled or disenabled. The apparatus may for example be designed to continue a measuring operation after being partly disenabled as a function of the marker or expiry signal, in order not to be started again after being stopped. In another embodiment, components of the apparatus may be enabled or disenabled. The components may for example be a control unit, controls, a pump for gas to be measured or a predetermined operating function.

In a preferred embodiment of the apparatus, the interval of time to expiry is represented by the item of marker information. In this way, a given water trap, having for example a predetermined volume of liquid, may advantageously have a predetermined period of use assigned to it.

In a preferred embodiment, the item of marker information represents a code which is assigned to a given water trap and in particular a predetermined water trap. The code may for example represent a date of manufacture, a serial number or a combination of these. In this way, it can advantageously be ensured that a water trap which is connected to the apparatus on the starting of the time counter has to be disconnected from the apparatus and replaced by another water trap. For this purpose, the apparatus may advantageously be designed to sense a code represented by the item of marker information and hold it stored in a memory. The apparatus may also be designed to interrupt a counting process by the time counter, and in particular the generation of the interval of time as a function of the marker signal, and to continue the generation of the interval of time on the marker signal being sensed again. With particular advantage, the apparatus may continue or restart the time counter as a function of the code represented by the item of marker information. For this purpose, the apparatus may compare the code sensed at the time of connection with the code previously stored and restart or continue the generation of the interval of time by the counting device as a function of the result of the comparison.

In a preferred embodiment, the water trap has a container for a liquid, in particular a hydrophilic one, and the radio-frequency marker is arranged inside the container and may be buoyant in the liquid which is in particular hydrophilic. The radio-frequency marker is preferably of a colour, and in particular a body colour, different from that of the liquid, and the container is transparent to electromagnetic radiation in the visible range at least in a region of a wall of the container. The radio-frequency marker may preferably be self-lit and for this purpose may for example have a light, and in particular a light-emitting diode, which shines through the wall of the container.

In the invention, the radio-frequency marker is designed to receive an item of marker information and to store it in such a way that it can be read out again. The radio-frequency sensing device is designed to transmit an item of marker information to the radio-frequency marker and to store it there. A period of time for which a given water trap has been connected to the apparatus can advantageously be sensed in this way. For this purpose, the apparatus may for example be designed to generate, before the water trap is disconnected from the apparatus, an item of marker information, in the form of a marker data set for example, which presents the interval of time generated by the time counter, and to transmit this item of marker information, i.e. the marker data set, to the radio-frequency marker and to store it there, by means of the radio-frequency sensing device, and in particular a read/write radio-frequency sensing device. If the water trap which was previously disconnected is connected again to the apparatus, the apparatus may for example sense the item of marker information, and in particular the period of connection represented by the interval of time, by means of the radio-frequency sensing device and may hold it in store in a memory.

As a further preference, the time counter may be designed to continue the counting of the period of connection as a function of the interval of time which is stored. In this way, a period for which a predetermined water trap has been connected may be sensed regardless of how often the predetermined water trap is disconnected from the apparatus and again continues to be operated with this apparatus or on another apparatus.

Preferred embodiments of a radio-frequency sensing device are designed to operate in a frequency range of 30 kilohertz to 500 kilohertz, in a frequency range from 800 to 950 megahertz or in a frequency range from 1 gigahertz to 3 gigahertz. The radio-frequency sensing device is preferably designed to operate at a frequency of 125 kilohertz or at a frequency of 13.56 megahertz. When transmitting a marker signal representing the item of marker information, the radio-frequency sensing device and the radio-frequency marker may for example operate by one of the following methods of modulation or by a combination of the following methods of modulation:
- FM (FM = frequency modulation),
- AM (AM = amplitude modulation),
- FSK (FSK = frequency shift keying),
- ASK (ASK = amplitude shift keying),
- PSK (PSK = phase shift keying).

The radio-frequency sensing device may preferably sense the radio-frequency marker by load modulation. For this, the radio-frequency sensing device may supply the radio-frequency marker with transmitting energy. For this purpose, the radio-frequency marker may for example have an energy store and may store the transmitting energy which is received and may use it to transmit back a transmitted response signal representing the item of marker information. What is also envisaged is an active radio-frequency marker which has an energy source for transmitting the item of marker information.

A water trap according to a further aspect of the present invention for a respirator apparatus may advantageously have a radio-frequency marker. The radio-frequency marker is advantageously arranged in the region of the water trap. It is also preferable for the radio-frequency marker to be coupled or connected to the water trap. For this purpose, the radio-frequency marker may for example be enclosed in a container belonging to the water trap, may be adhesive-bonded to the water trap or may be welded into a housing or a part of the water trap. The radio-frequency marker may preferably be buoyant in a liquid which is held in store by a container belonging to the water trap.

The invention also relates to a method of sensing a water trap which is disconnectably connected to an apparatus and which has a radio-frequency marker, comprising the steps of:
- sensing of the radio-frequency marker belonging to the water trap, and of an item of marker information represented by the radio-frequency marker, and generation of a marker signal representing the item of marker information,
- starting, as a function of the marker signal, of a time counter to increment a time value
- generation of an expiry signal as a function of the reaching of a predetermined time value, and control of the apparatus as a function of the expiry signal.

In an advantageous embodiment, the method comprises the step of:
- enablement or disenablement of components of the apparatus as a function of the expiry signal.

In an advantageous variant embodiment, the method comprises the step of:
- stopping or restarting or continuation of the counting by the time counter as a function of the marker signal.

The method also advantageously comprises the step of:
- transmission of the value of time to the radio-frequency marker and storage of the value of time in the radio-frequency marker, on the time counter being stopped.

The invention will now be explained in what follows by reference to drawings and to further embodiments.
Fig. 1 is a schematic view of an embodiment of respirator apparatus.
Fig. 2 is a schematic view of an embodiment of method for sensing a water trap which is disconnectably connected to a respirator apparatus and which has a radio-frequency marker.
Fig. 3 is a schematic view of an embodiment a water trap.
Fig. 4 is a schematic view of an embodiment of respirator system having the water trap shown in Fig. 3.

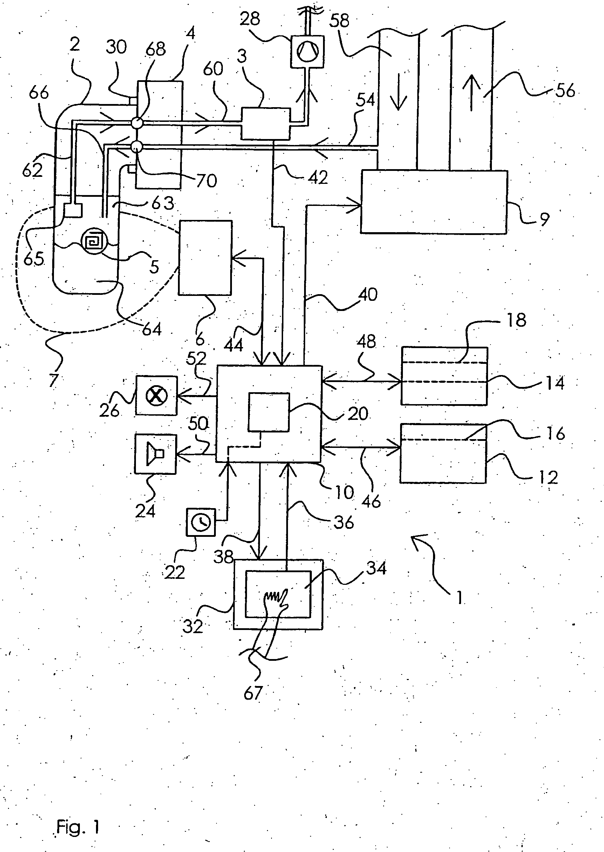

Fig. 1 is a schematic view of an embodiment of respirator apparatus 1. The respirator apparatus 1 has a water trap 2 which is designed for disconnectable connection to the respirator apparatus. The respirator apparatus 1 has for this purpose a receiving means 4 for connection to the water trap 2. The respirator apparatus 1 also has a gas sensor 3. The water trap 2 has a radio-frequency marker 5 which is enclosed in a container belonging to the water trap 2. The radio-frequency marker 5 is designed to be buoyant in a liquid, and in particular in a hydrophilic liquid which contains water for example. The respirator apparatus 1 also has a radio-frequency sensing device 6 which is designed to sense the radio-frequency marker 5 by load modulation and to generate a marker signal which corresponds to an item of marker information stored in the radio-frequency marker 5. The respirator apparatus 1 also has a respirator 9 which may take the form of a ventilating apparatus or an anaesthesia apparatus. The respirator apparatus 1 also has a central processing unit 10, a memory 12 and a memory 14. The memory 12 takes the form of a buffer memory and is designed to hold data sets stored, of which a data set 16 is identified by way of example. The memory 14 is designed to hold data sets stored, of which a data set 18 is identified by way of example. The respirator apparatus 1 also has a time counter 20 which in the present embodiment is part of the central processing unit 10. The time counter 20 is connected to a clock emitter 22 which is designed to generate a clock signal which forms a time-base. The clock emitter 22 may for example have a quartz oscillator to generate the clock signal. The respirator apparatus 1 also has a sound-generator 24 to generate an audio warning signal. The respirator apparatus 1 also has a signal light 26 which is designed to emit a light warning signal in the form of visible electromagnetic radiation, which is in particular visible for long distances. The respirator apparatus 1 also has a pump 28 which is connected on the input side to the gas sensor 3 and on the outlet side to, for example, the air surrounding the respirator apparatus 1. The water trap 2 is disconnectably connected to the receiving means 4 by means of a plug-in connection 30. For this purpose, the water trap 2 may have lug regions or projecting regions which may each be inserted into corresponding recesses in the receiving means 4, which recesses are designed to fit behind the lug regions or projecting regions of the water trap 2, in positive engagement therewith, in such way that the water trap 2 is releasably connected to the receiving means 4. For this purpose the lug regions or projecting regions of the water trap 2 and the recesses and projections of the receiving means 4 may be so formed and may be so arranged relative to one another that the water trap 2 can be inserted in the recesses in the receiving means 4 by for example being moved in translation along an axis of connection, and may be positively secured against disconnection along the axis of connection by then being moved in rotation about the axis of connection.

The respirator apparatus 1 also has an input unit 32 which is in the form of a display or image-reproducing unit and which has a touch-sensitive surface 34. The touch-sensitive surface 34 is designed to generate, in response to being touched, by the hand 67 of a user for example, a user-interaction signal which represents the point at which the touch-sensitive surface 34 is touched. The touch-sensitive surface is connected via a connecting conductor 36 to the central processing unit 10. The central processing unit 10 is connected on the output side via a connecting conductor 38 to the image-reproducing unit of the input unit 32. The central processing unit 10 is connected on the output side via a connecting conductor 40 to the respirator 9 and is designed to generate a control signal to control the respirator and to transmit this control signal via the connecting conductor 40 to the respirator 9.

The gas sensor 3 is connected on the output side via a connecting conductor 42 to the central processing unit 10 and is designed to detect, in particular photo-optically, and for example by infrared optics, and/or electrochemically a component gas in a flow of sample gas and to generate a sensor signal which represents the component gas. The component gas may for example be oxygen, carbon dioxide, nitrous oxide or anaesthetics. The central processing unit 10 is connected via a connecting conductor 46 to the memory 12 and via a connecting conductor 48 to the memory 14. The connecting conductors 46 and 48 are each of a bi-directional form and may for example each be formed by a bi-directional data bus.

The central processing unit 10 is connected on the output side via a connecting conductor 50 to the sound generator 24 and on the output side via a connecting conductor 52 to the signal light 26. The respirator 9 is connected on the outlet side for a gas flow to a ventilation line 56 which encloses a lumen to carry a gas. The respirator 9 is connected on the inlet side for the gas flow to a ventilation line 58. The ventilation line 58 encloses a lumen to carry a gas. The ventilation line 58 is connected to a sample-gas line 54 which encloses a lumen to carry the flow of sample gas. The lumen of the sample-gas line 54 is connected to the lumen of the ventilation line 58. The sample-gas line 54 connects the ventilation line 58 to a lumen belonging to the receiving means 4. The lumen of the receiving means 4, which is connected to the sample-gas line 54, has at the connection to the water trap 2 an opening 70 for connection to the water trap 2. The water trap 2 has a connecting line 66 which encloses a lumen to carry the sample gas. The sample-gas line 66 of the water trap 2 leads into a cavity 63 in the water trap 2 which is enclosed by a wall of the container belonging to the water trap 2. When the water trap 2 is in the state where it is connected to the receiving means 4, a sample-gas line 62 belonging to the water trap 2 connects the cavity 63 to the gas sensor 3 via an opening 68 in the receiving means 4, a lumen which is connected to the opening 68, and a sample-gas line 60 which encloses a lumen to carry the sample gas, which gas sensor 3 is connected on the input side to the sample-gas line 60. The sample-gas line 62 of the water trap 2 has, in the region of an end which opens into the cavity 63, a filter membrane 65 which is arranged between the lumen enclosed by the sample-gas line 62 and the cavity 63. The filter membrane 65 is permeable to gas and repellent of water. For this purpose the filter membrane may for example contain polytetrafluoroethylene. Illustrative embodiments of a filter membrane of this kind are a Goretex^{®} membrane or a Durapel^{®} membrane. The radio-frequency sensing device 6 is connected via a connecting conductor 44 to the central processing unit 10. The connecting conductor 44 is bi-directional in form.

The way in which components of the respirator apparatus 1 work and co-operate will now be explained in what follows:

The radio-frequency sensing device 6 will sense the radio-frequency marker of the water trap 2 when the radio-frequency marker is arranged within the sensing range 7. This means that the water trap 2 is connected to the receiving means 4. The radio-frequency sensing device 6 is able to read from the radio-frequency marker, by load modulation for example, and to generate a marker signal which represents an item of marker information relating to the radio-frequency marker 5. The item of marker information may be a date of production or a serial number or a combination of these. The radio-frequency sensing device is able to transmit the marker signal via the connecting conductor 44 to the central processing unit 10.

By means of the clock signal generated by the clock emitter 22 and the marker signal, the central processing unit 10 is able to generate a data set which represents the item of marker information, and in particular the serial number of the water trap 2, and a current date, namely the date when the water trap 2 is put into operation. As a function of the marker signal received via the connecting conductor 44, the central processing unit 10 is able to start the time counter 20, which is able to generate a time data set representing an interval of time.

The time counter 20 is able to increment the time data set as a function of the clock signal generated by the clock emitter 22. The time data set thus represents an interval of time which represents a period of time since the putting into operation of the water trap 2. The central processing unit 10 can then store the marker data set in the memory 14 via the connecting conductor 48. The data set 18 for example may form a marker data set. In the event of the water trap 2 being removed from the receiving means 4, and hence too of the radio-frequency marker 5 being removed from the sensing range 7 of the radio-frequency sensing device 6, the radio-frequency sensing device 6 may generate a stopping signal and may transmit this via the connecting conductor 44 to the central processing unit 10, or the central processing unit 10 may sense the absence of a marker signal which is generated by the radio-frequency sensing device 6 continually over time, at predetermined intervals for example, and may stop the time counter 20 as a function of the stopping signal or as a function of the absence of the marker signal, as the case may be. The central processing unit 10 may then generate a time data set which is assigned to the marker data set of the water trap 2, or a data set which represents the serial number of the water trap 2 and the period of time for which the water trap 2 has been connected to the receiving means 4, which is sensed by the time counter 20, and may store it in the memory 12 via the connecting conductor 46. The data set 16 is identified by way of example and may be a data set representing a serial number and a period of time connected.

Together, the memory 12 and the memory 14 may for example form a common memory, or together they may form a look-up memory. In a look-up memory, a marker data set which represents a serial number of a water trap is assigned to a time data set which represents a period of time connected.

In the event of the water trap 2 being re-connected to the receiving means 4, the radio-frequency sensing device 6 may sense the radio-frequency marker 5 of the water trap 2 and may generate a corresponding marker signal and may transmit this via the connecting conductor 44 to the central processing unit 10. The central processing unit 10 can then read the data set 16 from the memory 12 via the connecting conductor 46 and can continue to increment the interval of time represented by the data set 16 by means of the time counter 20 and can generate, for each step of the incrementation for example, a time data set which represents the incremented interval of time. The incremented time data set may for example be stored in the memory 12 via the connecting conductor 46 and held in store there. When there is a fresh incrementation operation by the time counter 20, the time data set can be read out of the memory 12 via the connecting conductor 46 and an incremented data set which represents a longer period of time connected may be stored afresh in the memory 12.

Before the respirator apparatus 2 is started, a maximum period of time for the water trap 2 to be connected to the receiving means 4 may be preset, via for example the input unit 32. For this purpose, the central processing unit 10 may generate, as a function of a user interaction signal received via the connecting conductor 36, a corresponding data set which represents a maximum period of time connected and may hold this in store in the memory 12. In another embodiment, the maximum period of time connected may for example be included in the marker information relating to the radio-frequency marker 5. In this embodiment, the marker signal may represent the maximum period of time connected of the water trap 2. The maximum period of time connected may be represented by for example a marker data set such for example as the data set 18. The central processing unit 10 may compare, at predetermined intervals of time for example, the time data set generated by the time counter 20 with the marker data set representing the maximum period of time connected and in this way may sense any overrun of the maximum period of time for which the water trap 2 may be connected to the receiving means 4 and thus to the respirator apparatus 1.

In the event of the maximum period of time connected being overrun, the central processing unit 10 can generate a warning signal and can transmit it via for example the connecting conductor 50 and/or the connecting conductor 52 to the sound generator 24 and/or the signal light 26.

Alternatively or in addition, the possibility exists of the overrun of the maximum period of time connected being indicated on the input unit 32.

In another embodiment, the central processing unit 10 may be designed to stop the pump 28 from operating as a function of an overrun of the maximum period of time for which the water trap 2 can be connected to the receiving means 4.

In another advantageous embodiment of the respirator 1, disconnection of the water trap 2 from the receiving means 4 can be initiated by a user interaction signal transmitted via the connecting conductor 36. For this purpose, the central processing unit 10 may, as a function of a user interaction signal of this kind, generate a marker data set which represents the period of time connected generated by the time counter 20 and may transmit it via the connecting conductor 44 to the radio-frequency sensing device 6. The radio-frequency sensing device 6 is able to transmit the marker data set to the radio-frequency marker 5 and store it there. In this embodiment, the radio-frequency sensing device is designed to write to and read from a radio-frequency marker. In this embodiment, the radio-frequency marker 5 is designed to be written to and read from. The radio-frequency marker 5 may be an active or passive radio-frequency marker. The radio-frequency marker 5 may advantageously be buoyant in a liquid and particularly in water or in a hydrophilic liquid 64. In the event of the container of the water trap 2 having a transparent wall, the level of the liquid 64 can be perceived in a visible form from outside from the local position of the radio-frequency marker 5 within the cavity 63 in the water trap 2. In this way, the radio-frequency marker 5 is able to perform two functions, namely indication of the level of the liquid 64 and holding in store of an item of marker information which is intended to be read by the radio-frequency sensing device 6.

When the water trap 2 is operating, the pump 28 at the output of the gas sensor 3 may generate a pressure below atmospheric and in this way, beginning from the lumen 58, may produce a flow of sample gas, via the sample-gas line 54, through the cavity 63 in the water trap 2, through the filter membrane 65 and, via the sample-gas line 62 and the sample-gas line 60 on the input side, to the gas sensor 3. The gas sensor 3 is able to generate a sensor signal as a function of the proportion which is sensed of a gas to be sensed and is able to transmit it via the connecting conductor 42 to the central processing unit 10. As a function of the sensor signal, the central processing unit 10 is able to generate a control signal to control the respirator 9 and is able to transmit it via the connecting conductor 40 to the respirator 9.

A respirator system may comprise the respirator apparatus 1 without the water trap 2, and the water trap 2. A water trap 2 for the respirator apparatus may have a radio-frequency marker.

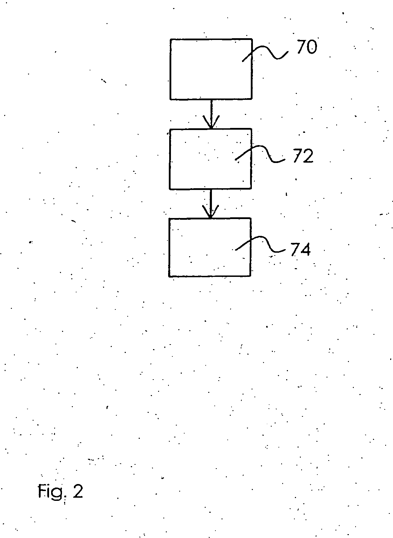

Fig. 2 is a schematic view of a method of sensing a water trap, having a radio-frequency marker, which is disconnectably connected to a respirator apparatus. In step 70, a radio-frequency marker belonging to the water trap, and an item of marker information represented by the radio-frequency marker, are sensed and a marker signal representing the item of marker information is generated. In step 72, a time counter which increments a time value is started as a function of the marker signal. In step 74, an expiry signal is generated as a function of a predetermined time value being reached and the respirator apparatus is controlled as a function of the expiry signal.

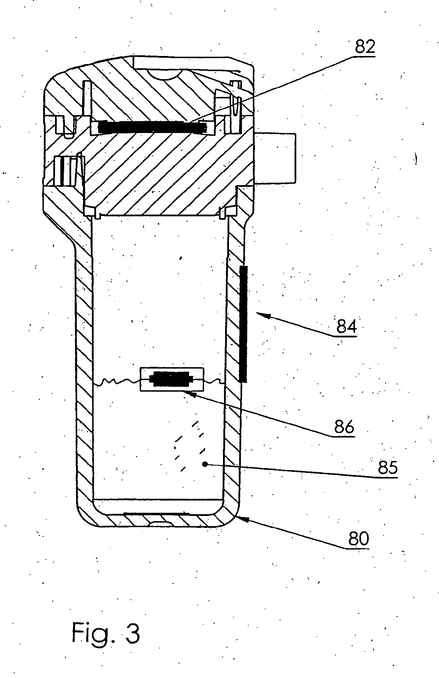

Fig. 3 is a schematic view of an embodiment of water trap 80. The water trap 80 has a radio-frequency marker 82 which is arranged in the head of the water trap 80 to be invisible from the exterior. In another embodiment, a radio-frequency marker is adhesive-bonded to the water trap. Fig. 3 also shows a radio-frequency marker 84 which is adhesive-bonded to the water trap 80. In another embodiment, a radio-frequency marker 86 is arranged in the water trap 80 and is designed to be buoyant in a liquid 85. The radio-frequency markers 82, 84 and 86 may each be implemented in or on a water trap independently of one another.

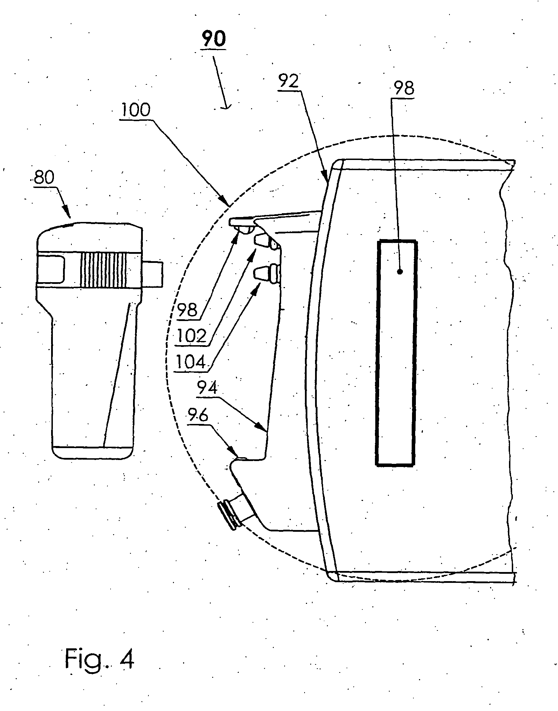

Fig. 4 is a schematic view of an embodiment of respirator system 90 having a respirator apparatus 92 and the water trap 80 which is shown in Fig. 3. The respirator apparatus 92 has the radio-frequency sensing device 6, which has a sensing range 100 for the radio-frequency marker 82, 84 or 86 and which is designed to sense the radio-frequency marker 82, 84 or 86 in the sensing range 100 and to generate a marker signal which represents an item of marker information relating to the radio-frequency marker 82, 84 or 86. The respirator apparatus 92 has a receiving means 94 for disconnectable connection to the water trap 80. The receiving means has for this purpose a lug 96, integrally formed on a projecting region, onto which a correspondingly formed region on the housing of the water trap 80 can be hooked. The receiving means 94 also has a finger 98 which is integrally formed to be pivotable on the receiving means 94 and which has a lug to engage in a corresponding recess in the water trap 80 and which is designed, having been pivoted into the recess in the housing of the water trap 80, to engage positively therein and to secure it against detachment. To allow it to pivot, the finger may have an appropriate modulus of elasticity or a hinging joint. The respirator apparatus 92 has a connection 102 for connection to a sample-gas line to feed gas in and a connection 104 for connection to a sample-gas line to feed gas away.

## Claims

1. An assembly including a water trap (2, 80) and an apparatus **characterized in that** the water trap (2, 80) has a radio-frequency marker (5, 82, 84, 86) which represents, as the item of marker information, an intended period of time for which the water trap may be used and in which the radio-frequency marker (5, 82, 84, 86) is arranged to receive the item of marker information and to store it in such a way that it can be read out again, and the apparatus includes a radio-frequency sensing device (6) arranged to transmit an item of marker information to the radio-frequency marker (5, 82, 84, 86).

2. The assembly of claim 1, in which the apparatus has a gas sensor (3) and is arranged to pass a flow of sample gas (54) through the water trap and to feed it to the gas sensor (3), the water trap (2, 80) being arranged to be disconnectably connected to the apparatus (1, 92) and the apparatus (1, 92) having a receiving means (4, 94) for connection to the water trap (2, 80), the radio-frequency sensing device (6) being arranged to sense the radio-frequency marker (5, 82, 84, 86) in a sensing range (7, 100) and to generate a marker signal which represents the item of marker information, and the apparatus (1, 92) is arranged to be controlled as a function of the marker signal.

3. The assembly according to claim 2, in which the apparatus has a time counter (20) which is connected at least indirectly to the radio-frequency sensing device (6) and which is arranged to generate, as a function of the marker signal, an interval of time which represents a period of time for which the water trap (2, 80) has been connected to the apparatus, and to compare the interval of time with a predetermined interval of time to expiry and to generate a result of the comparison and, as a function of the result of the comparison, to generate an expiry signal which represents an expiry of the predetermined interval of time to expiry, and the respirator apparatus is arranged to be controlled as a function of the expiry signal.

4. The assembly according to claim 2 or 3, in which the apparatus is arranged to be at least partly enabled or disenabled as a function of the marker signal.

5. The assembly according to either of claims 3 and 4, in which the interval of time to expiry is represented by the item of marker information.

6. The assembly according to one of the foregoing claims, in which the item of marker information relating to the radio-frequency marker (5, 82, 84, 86) represents a code which is assigned to the water trap (2, 80).

7. The assembly according to one of the foregoing claims, in which the water trap (2, 80) has a container for a liquid (64, 85), and the radio-frequency marker (5, 82, 84, 86) is arranged inside the container and may be buoyant in the liquid (64, 85).

8. A method of sensing a water trap which is disconnectably connected to an apparatus and which has a radio-frequency marker (5, 82, 84, 86), comprising the steps of:
a) sensing of the radio-frequency marker (5, 82, 84, 86) belonging to the water trap, and of an item of marker information represented by the radio-frequency marker, and generation of a marker signal (70) representing the item of marker information;
b) starting, as a function of the marker signal (72), of a time counter to increment a time value;
c) generating an expiry signal as a function of the reaching of a predetermined time value; and
d) controlling the apparatus as a function of the expiry signal (74).

9. The method according to claim 8, including the step of at least partial enablement or disenablement of the respirator apparatus as a function of the expiry signal.

10. The method according to claim 8 or 9, including the step of stopping or restarting of the time counter as a function of the marker signal.

11. The method according to claim 10, including the step of transmitting the value of time to the radio-frequency marker and storage of the value of time in the radio-frequency marker, on the time counter being stopped.

## Patentansprüche

1. Anordnung mit einer Wasserauffangvorrichtung (2, 80) und einer Einrichtung, **dadurch gekennzeichnet, dass** die Wasserauffangvorrichtung (2, 80) einen Hochfrequenzmarker (5, 82, 84, 86) aufweist, der als Markerinformation einen vorgesehenen Zeitraum darstellt, während dem die Wasserauffangvorrichtung verwendet werden kann, und wobei der Hochfrequenzmarker (5, 82, 84, 86) angeordnet ist, um die Markerinformation zu empfangen und diese derart zu speichern, dass sie wieder ausgelesen werden kann, und die Einrichtung eine Hochfrequenzdetektiervorrichtung (6), die angeordnet ist, um eine Markerinformation an den Hochfrequenzmarker (5, 82, 84, 86) zu senden.

2. Anordnung nach Anspruch 1, wobei die Einrichtung einen Gassensor (3) aufweist und angeordnet ist, um einen Strom von Probengas (54) durch die Wasserauffangvorrichtung hindurch zu führen und diesen dem Gassensor (3) zuzuführen, wobei die Wasserauffangvorrichtung (2, 80) angeordnet ist, um lösbar mit der Einrichtung (1, 92) verbunden zu werden, und die Einrichtung (1, 92) ein Aufnahmemittel (4, 94) zur Verbindung mit der Wasserauffangvorrichtung (2, 80) aufweist, wobei die Hochfrequenzdetektiervorrichtung (6) angeordnet ist, um den Hochfrequenzmarker (5, 82, 84, 86) in einem Detektierbereich (7, 100) zu detektieren und ein Markersignal zu erzeugen, welches die Markerinformation darstellt, und die Einrichtung (1, 92) angeordnet ist, um in Abhängigkeit von dem Markersignal gesteuert zu werden.

3. Anordnung nach Anspruch 2, wobei die Einrichtung einen Zeitzähler (20) aufweist, der mindestens indirekt mit der Hochfrequenzdetektiervorrichtung (6) verbunden ist und der angeordnet ist, um in Abhängigkeit von dem Markersignal ein Zeitintervall zu generieren, das einen Zeitraum darstellt, während dem die Wasserauffangvorrichtung (2, 80) mit der Einrichtung verbunden war, und um das Zeitintervall mit einem vorgegebenen Ablaufzeitintervall zu vergleichen und ein Ergebnis des Vergleichs zu erzeugen und in Abhängigkeit von dem Ergebnis des Vergleichs ein Ablaufsignal zu erzeugen, welches einen Ablauf des vorgegebenen Ablaufzeitintervalls darstellt, und das Beatmungsgerät angeordnet ist, um in Abhängigkeit von dem Ablaufsignal gesteuert zu werden.

4. Anordnung nach Anspruch 2 oder 3, wobei die Einrichtung angeordnet ist, um in Abhängigkeit von dem Markersignal mindestens teilweise freigegeben oder gesperrt zu werden.

5. Anordnung nach einem beliebigen der Ansprüche 3 und 4, wobei das Ablaufzeitintervall durch die Markerinformation dargestellt ist.

6. Anordnung nach einem der vorhergehenden Ansprüche, wobei die den Hochfrequenzmarker (5, 82, 84, 86) betreffende Markerinformation einen Code darstellt, welcher der Wasserauffangvorrichtung (2, 80) zugewiesen ist.

7. Anordnung nach einem der vorhergehenden Ansprüche, wobei die Wasserauffangvorrichtung (2, 80) einen Behälter für eine Flüssigkeit (64, 85) aufweist und der Hochfrequenzmarker (5, 82, 84, 86) innerhalb des Behälters angeordnet ist und in der Flüssigkeit (64, 85) schwimmen kann.

8. Verfahren zum Detektieren einer Wasserauffangvorrichtung, die lösbar mit einer Einrichtung verbunden ist und einen Hochfrequenzmarker (5, 82,84,86) aufweist, umfassend folgende Schritte:
a) Detektieren des zu der Wasserauffangrichtung zugehörigen Hochfrequenzmarkers (5, 82, 84, 86), und einer durch den Hochfrequenzmarker dargestellten Markerinformation, und Erzeugen eines Markersignals (70), das die Markerinformation darstellt;
b) Starten eines Zeitzählers in Abhängigkeit von dem Markersignal (72), um einen Zeitwert hochzuzählen;
c) Erzeugen eines Ablaufsignals in Abhängigkeit von dem Erreichen eines vorgegebenen Zeitwerts; und
d) Steuern der Einrichtung in Abhängigkeit von dem Ablaufsignal (74).

9. Verfahren nach Anspruch 8, umfassend den Schritt des mindestens teilweisen Freigebens oder Sperrens des Beatmungsgeräts in Abhängigkeit von dem Ablaufsignal.

10. Verfahren nach Anspruch 8 oder 9, umfassend den Schritt des Anhaltens oder neuerlichen Startens des Zeitzählers in Abhängigkeit von dem Markersignal.

11. Verfahren nach Anspruch 10, umfassend den Schritt des Sendens des Zeitwerts zu dem Hochfrequenzmarker und des Speicherns des Zeitwerts in dem Hochfrequenzmarker, nach Anhalten des Zeitzählers.

## Revendications

1. Ensemble comprenant un piège à eau (2, 80) et un appareil, **caractérisé en ce que** le piège à eau (2, 80) a un marqueur de radiofréquence (5, 82, 84, 86) qui représente, en tant qu'article de l'information du marqueur, un laps de temps souhaité pendant lequel le piège à eau peut être utilisé et dans lequel le marqueur de radiofréquence (5, 82, 84, 86) est disposé de façon à recevoir l'article d'information de marqueur et à le stocker de sorte qu'il puisse être relu, et l'appareil comprend un dispositif de détection de radiofréquence (6) disposé de façon à transmettre un article d'information de marqueur au marqueur de radiofréquence (5, 82, 84, 86).

2. Ensemble selon la revendication 1, dans lequel l'appareil a un capteur de gaz (3) et est disposé de façon à faire passer un flux de gaz échantillon (54) à travers le piège à eau et à l'amener au capteur de gaz (3), le piège à eau (2, 80) étant disposé de façon à être raccordé de manière déconnectable à l'appareil (1, 92) et l'appareil (1, 92) ayant un système de réception (4, 94) pour la connexion au piège à eau (2, 80), le dispositif de détection de radiofréquence (6) étant disposé de façon à détecter le marqueur de radiofréquence (5, 82, 84, 86) dans une gamme de détection (7, 100) et à générer un signal de marqueur qui représente l'article de l'information de marqueur, et l'appareil (1, 92) est disposé de façon à être contrôlé en fonction du signal de marqueur.

3. Ensemble selon la revendication 2, dans lequel l'appareil a un compteur (20) qui est connecté au moins indirectement au dispositif de détection de radiofréquence (6) et qui est disposé de façon à générer, en fonction du signal de marqueur, un intervalle de temps qui représente un laps de temps pendant lequel le piège à eau (2, 80) a été connecté à l'appareil, et à comparer l'intervalle de temps avec un intervalle prédéterminé de temps jusqu'à expiration et à générer un résultat de la comparaison et, en fonction du résultat de la comparaison, à générer un signal d'expiration qui représente une expiration de l'intervalle de temps prédéterminé jusqu'à expiration, et l'appareil respiratoire est disposé de façon à être commandé en fonction du signal d'expiration.

4. Ensemble selon les revendications 2 ou 3, dans lequel l'appareil est disposé de façon à être au moins partiellement activé ou désactivé en fonction du signal de marqueur.

5. Ensemble selon l'une quelconque des revendications 3 ou 4, dans lequel l'intervalle de temps jusqu'à expiration est représenté par l'article de l'information de marqueur.

6. Ensemble selon l'une des revendications précédentes, dans lequel l'article de l'information de marqueur relatif au marqueur de radiofréquence (5, 82, 84, 86) représente un code qui est attribué au piège à eau (2, 80).

7. Ensemble selon l'une des revendications précédentes, dans lequel le piège à eau (2, 80) est doté d'un récipient pour un liquide (64, 85) et le marqueur de radiofréquence (5, 82, 84, 86) est disposé à l'intérieur du récipient et peut flotter dans le liquide (64, 85).

8. Procédé de détection d'un piège à eau qui est raccordé de manière déconnectable à un appareil et qui est doté d'un marqueur de radiofréquence (5, 82, 84, 86) comprenant les étapes consistant à :
a) détecter le marqueur de radiofréquence (5, 82, 84, 86) appartenant au piège à eau et un article de l'information de marqueur représenté par le marqueur de radiofréquence, et la génération d'un signal de marqueur (70) représentant l'article de l'information de marqueur ;
b) démarrer, en fonction du signal de marqueur (72), un compteur de temps pour augmenter une valeur de temps.
c) générer un signal d'expiration en fonction de la réalisation d'une valeur de temps prédéterminée ; et
d) commander l'appareil en fonction du signal d'expiration (74).

9. Procédé selon la revendication 8, comprenant l'étape consistant à activer ou désactiver au moins partiellement l'appareil respiratoire en fonction du signal d'expiration.

10. Procédé selon les revendications 8 ou 9, comprenant l'étape consistant à arrêter ou redémarrer le compteur en fonction du signal de marqueur.

11. Procédé selon la revendication 10, comprenant l'étape consistant à transmettre la valeur de temps au marqueur de radiofréquence et le stockage de la valeur de temps dans le marqueur de radiofréquence, à l'arrêt du compteur.
